# EUROPEAN PATENT SPECIFICATION

(11) **EP 3 426 340 B1**
(45) Date of publication and mention of the grant of the patent: **12.05.2021**
(21) Application number: 17763893.9
(22) Date of filing: 07.03.2017
(51) Int. Cl.: A61N 5/04, A61N 5/06, A61L 2/02, A61L 2/08, A61L 2/10

(54) **DEVICES REGULATING THE CIRCADIAN CYCLE**
VORRICHTUNGEN ZUR REGELUNG DES BIORHYTMUS
DISPOSITIFS DE RÉGULATION DU CYCLE CIRCADIEN

(30) Priority: 08.03.2016 US 201662305229 P; 16.09.2016 US 201662395700 P; 30.01.2017 US 201762451815 P
(43) Date of publication of application: 16.01.2019
(73) Proprietor: Signify North America Corporation, Somerset, NJ 08873 (US)
(72) Inventor: Grajcar, Zdenko, Orono, MN 55359 (US); Stephan, Aaron, Chanhassen, MN 55317 (US)
(74) Representative: Verweij, Petronella Daniëlle
(86) International application number: PCT/US2017/021137
(87) International publication number: WO 2017/155982

(56) References cited:
- WO-A1-2015/184019
- RU-C2- 2 295 989
- US-A- 4 109 414
- US-A1- 2008 266 690
- US-A1- 2010 076 250
- US-A1- 2010 179 469
- US-A1- 2013 165 741
- US-A1- 2014 062 297
- US-A1- 2014 067 130
- US-A1- 2014 228 914
- US-A1- 2016 023 017

## Description

### TECHNICAL FIELD

This document pertains generally, but not by way of limitation, to regulating the circadian cycle. More specifically this document relates to devices and methods used to regulate the circadian cycle.

### BACKGROUND

The circadian cycle is a biological process animals and plants display over a 24-hour cycle to define when an individual sleeps and is awake, and when a plant rests. Circadian rhythms are considered endogenous or self-sustained, and adjust to environmental factors such as light, temperature and redox cycles. Specifically, the body secrets hormones that regulate when an individual feels tired and desires to sleep, and when individuals are awake. Two such hormones are cortisol and melatonin that can have an inverse relationship to one another, where typically melatonin increases at night in order to cause an individual to become sleepy and fall asleep.

Many external factors can cause disruption to an individual's circadian cycle. For example, when an individual moves across many time zones, the body continues to produce melatonin and other hormones on the 24-hour schedule prior to travel, even though environmental conditions such as light and dark have changed. This is commonly referred to as jet lag. With a jet lagged individual, the body recognizes or reacts to the environmental changes and over time, delays or speeds up hormone production such as melatonin production to regulate the circadian cycle so that hormones are secreted at appropriate times by the body to match the external environment. Once the adjustment period is complete the individual's circadian cycle matches with the external environment as though the travel had not occurred.

Other external factors can also cause disruptions to an individual's circadian cycle, including, but not limited to, stress, head trauma, stroke, insomnia and the like. In all conditions, either improper amounts of hormones such as melatonin or cortisol are secreted or hormones are secreted at improper time intervals disrupting the circadian cycle and preventing normal sleep cycles. When normal sleep cycles are prevented, negative effects can result for the individual, including but not limited to weight gain, lack of concentration, moodiness, depression, immune system deficiencies and the like. Thus, a need exists to regulate the circadian cycle to assist the body in regulating the circadian cycle when disruptions occur.

Similarly, the circadian cycle and circadian rhythm is instrumental in both plant and animal growth. Disruptions in the circadian cycle, similarly cause health, growth, immune system and other deficiencies in both plants and animals. Thus a need exists for a system that can be utilized to better regulate the circadian cycle in plants, animals and humans.

Research has been conducted in mice showing that light absorbed in the retina of mice can be utilized to entrain circadian rhythms. Specifically, neuropsin (OPN5), a gene encoded in protein within the retina absorbs light resulting in the biological response of resetting a mammal's circadian rhythm. In particular, by eliminating neuropsin, while a circadian rhythm existed within the eye of the mice, the mice were unable to adjust their circadian rhythm to account for phase shifts in periods of light and dark as compared to mice where the circadian rhythm was reset. In this context, reference is made to documents US2008/266690 A1 and US4,109,414 A. Z

### SUMMARY

The invention is defined in the appended set of claims. The present inventors have recognized, among other things, that light sources for humans that assist in the regulation of the circadian cycle are lacking, and that the present subject matter can help provide a system to assist plants, animals and humans in regulating their circadian cycle.

In summary, a system for entraining a circadian rhythm of a living organism by providing light substantially concentrated in a narrow band of wavelengths centered about a predetermined wavelength that is absorbed by neuropsin is disclosed herein. A lighting system is provided that has at least one lighting element emitting light substantially concentrated in the narrow range between 360 nanometers (NM) and 400 NM to be absorbed by the neuropsin. A controller has a timing mechanism to provide the light substantially concentrated in the narrow range between 360 NM and 400 NM at intervals during multiple periods during a day. The controller is configured to set the intervals during the day based on a local time of day to entrain the circadian rhythm of the living organism.

This overview is intended to provide an overview of subject matter of the present patent application. It is not intended to provide an exclusive or exhaustive explanation of the invention. The detailed description is included to provide further information about the present patent application

### BRIEF DESCRIPTION OF THE FIGURES

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Fig. 1 is a schematic of a system for entraining a circadian rhythm of a living organism.
Fig. 2 depicts a person utilizing a circadian cycle regulating system.
Fig. 3 depicts a circadian cycle regulating system.
Fig. 4 is a schematic of a system for entraining a circadian rhythm of a living organism.
Fig. 5 is a schematic of a controller for a lighting device.
Fig. 6 is a flow diagram of a method to entrain a circadian rhythm.

### DETAILED DESCRIPTION

Light from the sun is generally shown to regulate circadian rhythm within humans. In addition, the body naturally produces vitamin D that regulates protein production increasing or decreasing gene production. To that end 1α, 25-(OH)2D3 entrains circadian rhythms in cell cultures. In particular, 1α,25-(OH)2D3 is able to synchronize circadian clock gene expression in adipose-derived stem cells (ADSC).

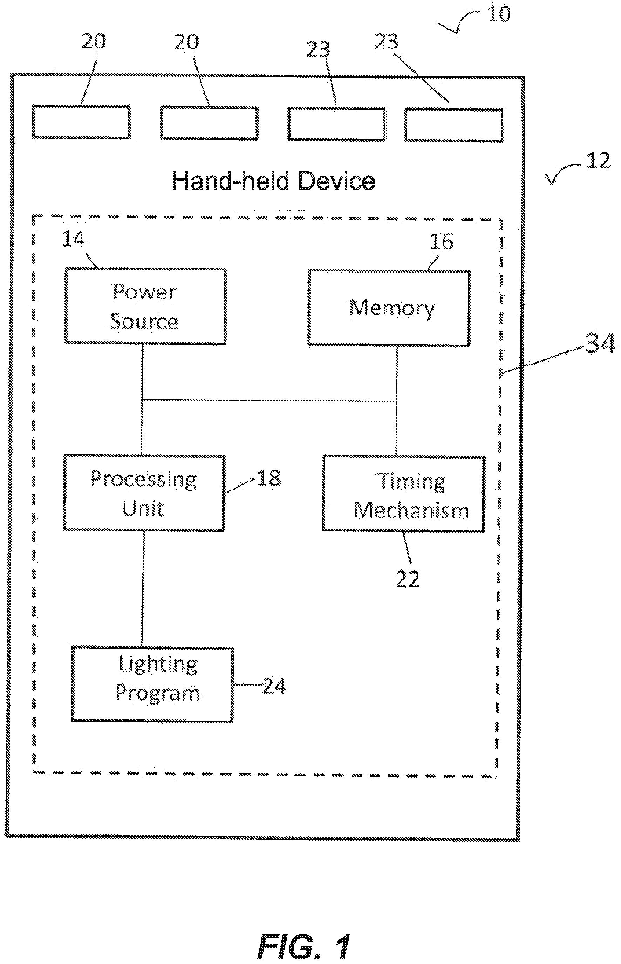

Fig. 1 illustrates a circadian cycle regulating system 10 that includes a hand-held device 12 for entraining a circadian rhythm of a living organism. The hand-held device 12 can be any electronic device that an individual can carry with them, including, but not limited to, a tablet, a smartphone, a mobile phone, a wireless headset, a PDA, a watch, a laptop computer or the like that has a power source 14 such as a battery, solar cell, or the like.

The hand-held device 12 includes a memory 16 and a processing unit 18 that can execute computer readable instructions. The processing unit 18 may include, or be connected to a radio receiver that is capable of over the air communication and receives signals from other electronic devices, such as through Wi-Fi or cellular transmissions, or the like. Electrically connected and actuated by the power source 14 is at least one lighting element 20. More than one lighting element 20 can be included on the hand-held device 12.

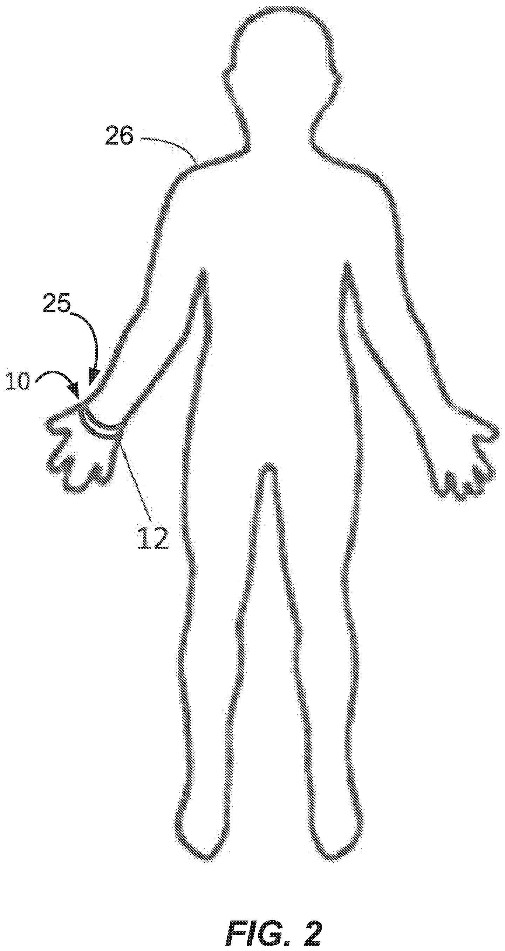

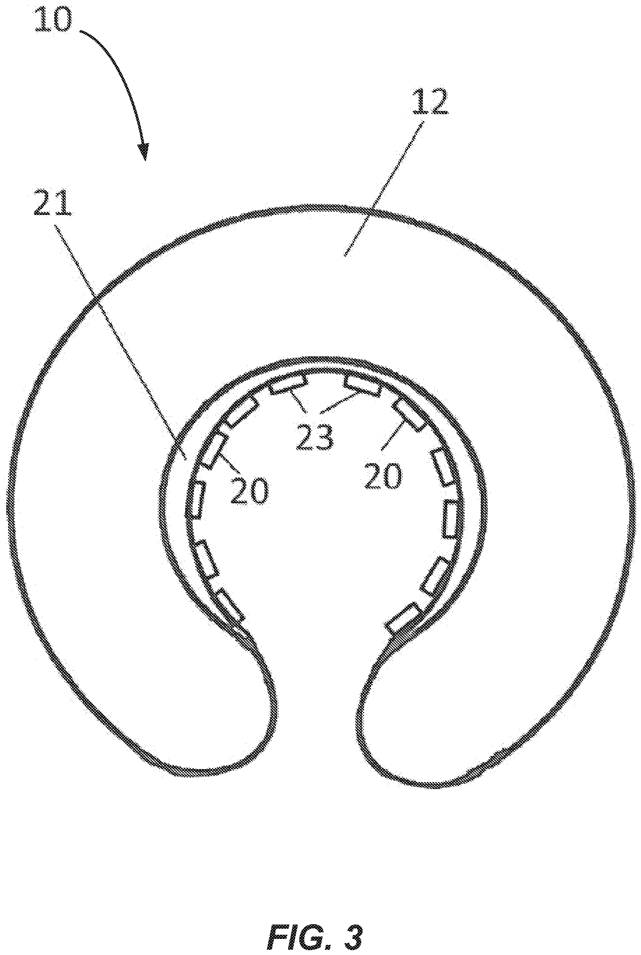

As depicted in Figs. 2 and 3, the hand-held device 12 can include, but is not limited to, a watch, bracelet, or band utilizing a flexible circuit board 21 that includes the power source 14, such as driving circuitry, and lighting elements 20 that are worn around the wrist 25 of a user 26. In this manner the lighting elements 21 are placed adjacent to, and in one example, engaging the skin of the user 26 such that the lighting elements 20 emit the light of the lighting element 20 (or elements 23, 32a or 32b) onto the skin or wrist of the individual wearer. While shown and described as a hand-held device 12, additional devices, such as laptop computing devices, televisions, additional electronic equipment often used by humans is contemplated without falling outside the scope of this disclosure.

At least one lighting element 20 is powered by the power source 14 in any way known in the art, including but not limited to an embodiment where the lighting element 20 is a light emitting diode (LED). In one example, the lighting element 20 emits UV light, specifically light at a wavelength that is less than 400 NM. In particular UV radiation in a range that suppresses the output of melatonin, but at a low level of intensity, for example less than 3 lux such that the radiation does not harm an individual when individual receives the radiation emitted by the lighting element 20 for a prolonged period of time.

In an example, the lighting element or elements 20 emit light substantially concentrated within a narrow range of wavelengths between 295 NM - 325 NM and preferably 309 NM. Note that a light source or lighting element is operative to produce light having a spectrum substantially concentrated within the specified range of wavelength (e.g., 295 NM - 325 NM) when over 90% or over 95% of the lighting energy emitted by the light source is within the specified narrow range of wavelengths. In some examples, the light source may thus also emit a small amount of light (e.g., less than 10%, or less than 5% of lighting energy) outside of the specified range.

In particular, 1α, 25-(OH) 2D3 or vitamin D, and specifically D3, absorbs 309nm light for vitamin D synthesis to entrain circadian rhythms in cell cultures. In particular, 1α,25-(OH)2D3 synchronizes circadian clock gene expression in ADSC (adipose-derived stem cells). By supplementing and enhancing this proper gene expression the circadian cycle is balanced and negative health effects such as jet-lag, hypertension, obesity and the like are minimized or eliminated. Thus, by providing doses of UV light in a narrow range of wavelengths based on the absorption peak of vitamin D3, or approximately at and around a 309 NM wavelength, preferably at an intensity level that does not result in the burning of the skin due to UV radiation exposure, the health of the individual is improved.

In one example, the light substantially concentrated within the narrow range causes a biological reaction in neuropsin (OPN5) within the retina, such as being absorbed by the neuropsin. Specifically, neuropsin is shown to absorb UVA wavelengths (315 NM - 400 NM) and preferably between 360 NM - 400 NM. As a result of utilizing the narrow range of UVA wavelengths by the lighting element 20, circadian rhythms of a mammal are photoentrained to reduce problems and detrimental effect caused by improper photoentrainment of an individual's circadian rhythm.

While described as at least one lighting element 20, the hand-held device 12 can have a plurality of lighting elements, including a second lighting element 23 that produces a different wavelength of radiation or light. In addition, while UV radiation is described as emitted other wavelengths in the visible and infrared wavelength range, including but not limited to blue wavelengths are contemplated by this disclosure.

A timing mechanism 22 is in communication with and is electrically connected to the processing unit 18 to control the actuation of the lighting element 20. The timing mechanism 22 in one embodiment is on a 24-hour cycle and actuates the lighting element 20 for predetermined periods of time during predetermined intervals. In one example, the predetermined period is ten minutes and the predetermined interval is three hours. Thus, every three hours the timing mechanism 22 actuates the lighting element 20 for ten minutes to emit light or radiation.

In particular, a lighting program 24 can be provided in the memory 16 such that the predetermined periods happen at predetermined times during a 24-hour cycle. In one example the predetermined periods of light or radiation occur at 9:00 A.M., noon, 3:00 P.M. and 6:00 P.M. In another example the predetermined periods of light or radiation occur at 10:00 A.M., noon, 2:00 P.M., 4:00 P.M. and 6:00 P.M. In addition, because the hand-held device 12 is in over the air communication to receive electronic signals, the hand-held device can adjust the time as an individual goes through different time zones as a result of communication with a network time service, or a global positioning system connected to or included within the processing unit 18. Thus, the predetermined periods of light or radiation occur based on the local time, not a preset 24-hour cycle. Further, the timing mechanism prevents the UV light from being emitted after a certain predetermined local time such as 7:00 PM to prevent altering the normal circadian rhythm of the user.

In operation an individual has a hand-held device 12 that emits radiation or light from its lighting element 20 that either suppresses or enhances production of a specific hormone, such as melatonin at predetermined intervals. In an example where melatonin is suppressed, an individual's circadian rhythms are determined and during a time when melatonin should not be within an individual's system the hand-held device 12 emits the light or radiation for a predetermined period at an intensity to cause suppression of the melatonin for a predetermined period.

Then the duration of suppression is determined and before melatonin can improperly start producing again, the lighting element 20 again emits light or radiation to suppress the melatonin production for another predetermined period of time. This is repeated consistent with the program until melatonin production is desired. At this point the lighting element 20 is no longer actuated and in one embodiment a second lighting element 23 provides a second wavelength of light or radiation that enhances melatonin production. In an example, the second wavelength of light or radiation is a red wavelength.

Therefore, when an individual travels over many time zones on a plane the timing mechanism 22 of the hand-held device 12 receives signals to reset the time displayed and provided by the timing mechanism 22. The program 24 then is utilized to cause the emission of radiation or light at predetermined intervals based on the specific time of the timing mechanism and not on a 24-hour cycle. In this manner melatonin is suppressed at times when it should be suppressed in the circadian cycle as a result of the radiation or light emitted by the lighting element 20 of the hand-held device 12 to supplement the body adjusting to new environmental conditions. Thus the resetting of the circadian cycle is reset faster than without use of the radiation or light.

Similarly, when someone is experiencing increased hormone secretion at inappropriate times through a 24-hour cycle as a result of stress, head trauma, stroke or the like, the lighting element 20 provides the light or radiation to supplement and further regulate the hormone secretion to provide proper levels throughout a 24-hour cycle. As a result, an individual's hormones are regulated to reduce the risk of negative health effects as a result of improper hormone levels, including but not limited to depression, moodiness or mood swings, lack of concentration, increased immune deficiencies and the like. Thus all of the problems discussed above have been overcome.

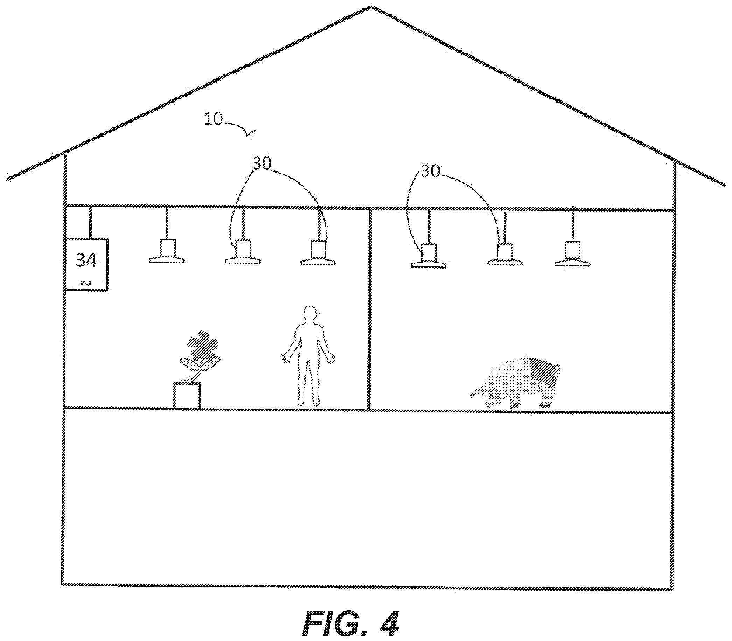

While described in association with humans, similarly when lighting both plants and animals, the circadian cycle can be regulated through proving predetermined periods of radiation or light at predetermined intervals to either suppress or enhance predetermined hormone or biological secretions, even though plants do not have neuropsin. By providing the periodic doses of radiation or light the circadian cycle is regulated. In such an embodiment as provided in Fig. 4, a plurality of lighting devices 30 are provided with each lighting device 30 having lighting elements 32a and 32b that emit radiation or light at predetermined wavelengths, such as under 400 NM, in the blue range of wavelengths or within the red range of wavelengths. These type of lighting devices 30 similarly can be utilized on humans, but is described in the embodiments as utilized on plants and animals for exemplary purposes only.

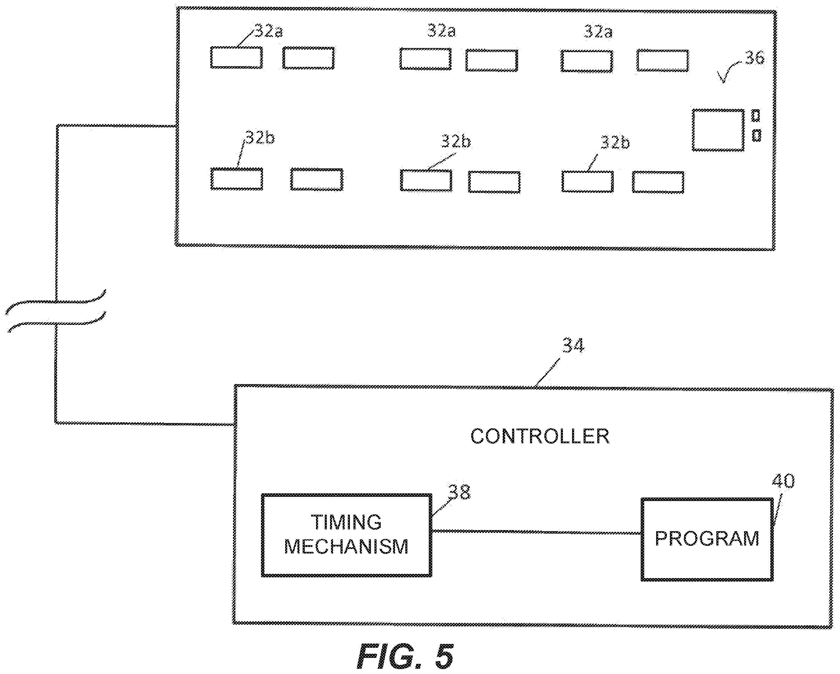

A controller 34, as shown in Fig. 5, is provided that in an embodiment includes a dimming device that actuates the lighting elements to emit radiation or light. In an example each lighting device 30 has sets of lighting elements 32a and 32b that are light emitting diodes with each set having a different lighting output. Fig. 5 shows a substrate of the lighting devices 32 that has sets of the lighting elements 32a and 32b and placed within the lighting device as is known in the art.

In an example, the first set of lighting elements 32a produce white, full spectrum light, the second set of lighting elements 32b produce UV light, or light having a wavelength less than 400 NM. These sets of lighting elements 32a and 32b are actuated by driving circuitry 36 such that at full intensity both the first and second set of lighting elements 32a and 32b emit radiation and/or light and as the lighting devices 30 are dimmed by the controller 34 the second set of lighting elements 32b that is emitting UV light stops emitting light so only full spectrum light is emitted. In this manner the controller disproportionately controls the different lighting elements such that the intensity of the first lighting elements 32a decreases at a faster rate than the second lighting elements 32b. For that matter, the first lighting elements 32a turn off and stop emitting light while the second lighting elements 32b continue emitting light.

Examples of lighting devices 32 and driving circuitry 36 that is able to operate in a manner to turn off a set of lighting elements while continuing operation of other lighting elements can be seen in at least U.S. application serial number 14/906,685, filed Jan. 21, 2016, among other patents and patent applications filed by Applicant.

In this manner the controller 34 utilizes a timing mechanism 38 and program 40 as previously provided to provide light from the lighting devices 30 at a predetermined dimmer level or intensity that results in the second set of lighting elements 32b not to emit UV radiation. Then for predetermined periods during predetermined intervals as provided by the program 40 the controller 34 is actuated to actuate the second set of lighting elements 32b to emit the UV radiation during the predetermined period at an intensity such that when the radiation reaches the animal or plant the radiation is not at an intensity that harms the plants or animals. Instead the radiation causes a predetermined biological response, such as suppressing melatonin. Thus, the circadian cycle of the animal or plant is regulated improving the health and wellbeing of the plant or animal. While described as having first and second lighting elements 32a and 32b, additional lighting elements that are different wavelengths can be added to the lighting device without falling outside the scope of this disclosure.

As a first example of a system for entraining a circadian rhythm of a human, a hand-held device is provided that has a lighting system with at least one lighting element. In this example the hand-held device is a bracelet worn by a person and the lighting element is a light emitting diode. The bracelet includes a controller that is configured to set intervals of 10 minutes of radiation emitted at a wavelength substantially concentrated at 390 NM starting at 8:00 A.M. and every 2 hours thereafter until 6:00 P.M. Radiation is then no longer emitted by the lighting element until 8:00 A.M. the next day.

In a second example a system for entraining a circadian rhythm of an animal, a lighting system is provided in an agricultural facility that has lighting devices that have a plurality of lighting elements that emit light at a predetermined range of wavelengths or predetermined color temperature onto avian. The lighting devices also have auxiliary lighting elements that emit light substantially concentrated at 380 NM. The lighting devices are electrically connected to a dimming device that operates as a controller. The dimming device is programed to increase the voltage to the lighting devices at 9:00 A.M. causing the lighting elements that emit light substantially concentrated at 380 NM to emit light for a period of 15 minutes. After the 15 minutes the dimming device reduces the voltage back to the level prior to the voltage increase, resulting in the lighting element that emit light substantially concentrated at 380 NM to stop emitting light. The dimmer is similarly programed to increase and decrease voltage at noon, 3 P.M. and 6 P.M. before all of the dimming device turns all of the lighting devices off until the next morning.

In another example a system for entraining a circadian rhythm of a plant, a lighting system is provided in a horticultural facility that has lighting devices that have a plurality of lighting elements that emit light at a predetermined range of wavelengths or predetermined color temperature onto plants. The lighting devices also have auxiliary lighting elements that emit light substantially concentrated at 390 NM. The lighting devices are electrically connected to a dimming device that operates as a controller. The dimming device is programed to increase the voltage to the lighting devices every hour, causing the lighting elements that emit light substantially concentrated at 380 NM to emit light for a period of five minutes at which time the voltage is decreased to stop the auxiliary lighting elements form emitting light. This occurs every hour for an entire 24-hour cycle.

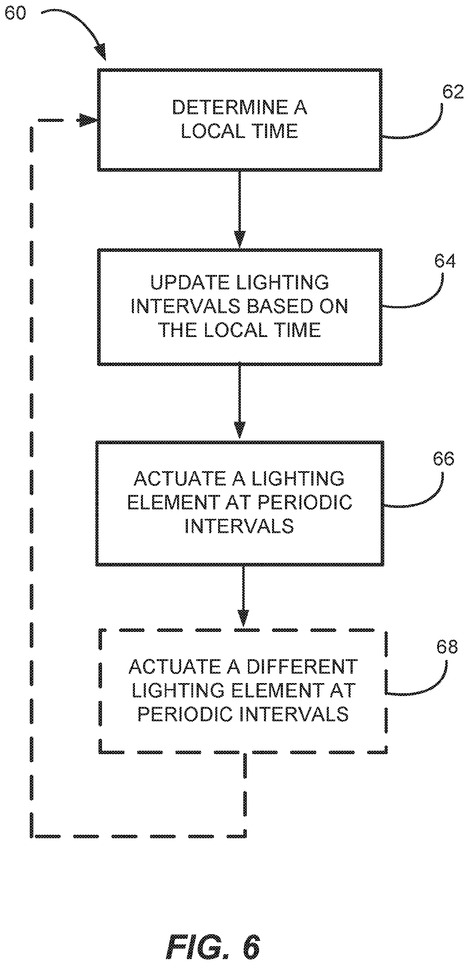

Fig. 6 is a flow diagram of a method 60 to entrain a circadian rhythm. A controller can be used to determine a local time 60. The local time may be based on received GPS information or time and location information received over a wired or wireless network. At 64 the controller can update one or more lighting intervals based on the local time. At 66 on or more lighting elements are actuated at periodic intervals according to the local time. Optionally, at 68 a second lighting element can be actuated by the controller during a second periodic interval according to the local time.

As another example of a system for entraining a circadian rhythm of a human, a laptop computer is provided that has a lighting system with at least one lighting element. The computer has a processor that is configured to set intervals of ten minutes of radiation emitted at a wavelength substantially concentrated at 390 NM starting at 8:00 A.M. and every two hours thereafter until 6:00 P.M. Radiation is then no longer emitted by the lighting element until 8:00 A.M. the next day. The processor is in communication with a global positioning system such that the processor determines when the computer enters different time zones and is configured to adjust or reconfigure the times the lighting element emits light to be at the predetermined times such as 8:00 AM, 10:00 AM and the like for the specific time zone the computer is located.

In an example embodiment a system for entraining a circadian rhythm of a living organism is provided. The system includes a lighting system having at least one lighting element emitting light substantially concentrated in a narrow range between 360 NM - 400 NM. The system also includes a controller having a timing mechanism configured to provide the light substantially concentrated in the narrow range between 360 NM - 400 NM at intervals during a day. The embodiment also provides that the controller is configured to set the intervals during the day based on a local time of day to entrain the circadian rhythm of the living organism. In this manner the system can help to adjust the circadian rhythm of a user from a first time zone to a second time zone.

In an example embodiment at least one lighting element is a light emitting diode. In another embodiment the lighting system is on a hand-held device. In this embodiment the hand-held device is a phone or a watch.

In an example embodiment the living organism is a human. In another embodiment the living organism is a plant. In yet another embodiment the living organism is an animal.

In an example embodiment the controller has a processing unit with a global positioning system and is configured to reset the intervals during the day based on information received from the global positioning system. In another embodiment the controller is configured to prevent emission of light by the at least one lighting element after a predetermined time during the day. In yet another embodiment the day is a 24-hour cycle.

Thus presented are lighting systems that utilize narrow bands of wavelengths of light to cause predetermined biological response to assist in regulating the circadian cycle. By regulating the circadian cycle health and quality of life is improved and thus all of the problems outlined in the background are overcome. By regulating the circadian cycle and thus hormone secretion, the lighting can be used in commercial settings such as shopping facilities, casinos, hospitals, or the like to regulate the mood, health and feeling of the individual under the lighting devices 30. In this manner the lighting devices 30 promote commercial activity of the users in one embodiment.

A number of implementations have been described. Nevertheless, it will be understood that various modification may be made. For example, advantageous results may be achieved if the steps of the disclosed techniques were performed in a different sequence, or if components of the disclosed systems were combined in a different manner, or if the components were supplemented with other components. Accordingly, other implementations are within the scope of the following claims.

The above description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims.

## Claims

1. A system for entraining a circadian rhythm of a living organism , comprising:
a lighting system having at least one lighting element emitting light substantially concentrated in a range between 360 nm and 400 nm, and
a controller having a timing mechanism configured to provide the light substantially concentrated in the range between 360 nm and 400 nm at intervals during a day;
wherein the controller is configured to set the intervals during the day based on a 24-hour schedule to entrain the circadian rhythm of the living organism; **characterized in that** the controller includes a processing unit with a global positioning system, and the processing unit is configured to adjust the 24-hour schedule and the intervals during the day based on information received from the global positioning system.

2. A system for entraining a circadian rhythm of a living organism , comprising:
a lighting system having at least one lighting element emitting light substantially concentrated in a range between 360 nm and 400 nm; and
a controller having a timing mechanism configured to provide the light substantially concentrated in the range between 360 nm and 400 nm at intervals during a day;
wherein the controller is configured to set the intervals during the day based on a 24-hour schedule to entrain the circadian rhythm of the living organism; **characterized in that** the controller includes a receiver unit configured to determine a local time from information received from a network time service; and the controller is configured to adjust the 24-hour schedule and the intervals during the day based on the local time.

3. A system for entraining a circadian rhythm of a living organism , comprising:
a lighting system having at least one lighting element emitting light substantially concentrated in a range between 360 nm and 400 nm; and
a controller having a timing mechanism configured to provide the light substantially concentrated in the range between 360 nm and 400 nm at intervals during a day;
wherein the lighting system is a hand- held device, **characterized in that** the 24-hour schedule is adjusted in response to a movement of the hand-held device to a new time zone.

4. The system of claim 1-3 , wherein the at least one lighting element is a light emitting diode.

5. The system of claims 1-3 , being suitable for entraining the circadian rhythm of a
human, wherein the controller is configured to set the intervals during the day based on a 24-hour schedule to entrain the circadian rhythm of the human.

6. The system of claim 1-3 , being suitable for entraining the circadian rhythm of a
plant, wherein the controller is configured to set the intervals during the day based on a 24-hour schedule to entrain the circadian rhythm of the plant.

7. The system of claim 1-3 , being suitable for entraining the circadian rhythm of an
animal, wherein the controller is configured to set the intervals during the day based on a 24-hour schedule to entrain the circadian rhythm of the animal.

8. The system of claim 1-3 , wherein the controller is configured to prevent emission
of light by the at least one lighting element after a predetermined time during the day.

## Patentansprüche

1. System zum Synchronisieren eines circadianen Rhythmus eines lebenden Organismus, umfassend:
- ein Beleuchtungssystem, das mindestens ein Beleuchtungselement aufweist, das Licht im Wesentlichen konzentriert in einem Bereich zwischen 360 nm und 400 nm emittiert, und
- eine Steuereinheit, die einen Zeitgebermechanismus aufweist, die konfiguriert ist, um das Licht im Wesentlichen konzentriert in dem Bereich zwischen 360 nm und 400 nm in Intervallen während eines Tages bereitzustellen;
- wobei die Steuereinheit konfiguriert ist, um die Intervalle während des Tages auf Grundlage eines 24-Stunden-Zeitplans einzustellen, um den circadianen Rhythmus des lebenden Organismus zu synchronisieren;
- **dadurch gekennzeichnet, dass** die Steuereinheit eine Verarbeitungseinheit mit einem globalen Positionierungssystem beinhaltet, und die Verarbeitungseinheit konfiguriert ist, um den 24-Stunden-Zeitplan und die Intervalle während des Tages auf Grundlage von Informationen anzupassen, die von dem globalen Positionierungssystem empfangen werden.

2. System zum Synchronisieren eines circadianen Rhythmus eines lebenden Organismus, umfassend:
- ein Beleuchtungssystem, das mindestens ein Beleuchtungselement aufweist, das Licht im Wesentlichen konzentriert in einem Bereich zwischen 360 nm und 400 nm emittiert; und
- eine Steuereinheit, die einen Zeitgebermechanismus aufweist, die konfiguriert ist, um das Licht im Wesentlichen konzentriert in dem Bereich zwischen 360 nm und 400 nm in Intervallen während eines Tages bereitzustellen;
- wobei die Steuereinheit konfiguriert ist, um die Intervalle während des Tages auf Grundlage eines 24-Stunden-Zeitplans einzustellen, um den circadianen Rhythmus des lebenden Organismus zu synchronisieren;
- **dadurch gekennzeichnet, dass** die Steuereinheit eine Empfangseinheit beinhaltet, die konfiguriert ist, um eine Ortszeit aus Informationen zu ermitteln, die von einem Netzwerkzeitdienst empfangen werden; und die Steuereinheit konfiguriert ist, um den 24-Stunden-Zeitplan und die Intervalle während des Tages auf Grundlage der Ortszeit anzupassen.

3. System zum Synchronisieren eines circadianen Rhythmus eines lebenden Organismus, umfassend:
- ein Beleuchtungssystem, das mindestens ein Beleuchtungselement aufweist, das Licht im Wesentlichen konzentriert in einem Bereich zwischen 360 nm und 400 nm emittiert; und
- eine Steuereinheit, die einen Zeitgebermechanismus aufweist, die konfiguriert ist, um das Licht im Wesentlichen konzentriert in dem Bereich zwischen 360 nm und 400 nm in Intervallen während eines Tages bereitzustellen;
- wobei das Beleuchtungssystem eine tragbare Vorrichtung ist, **dadurch gekennzeichnet, dass** der 24-Stunden-Zeitplan als Antwort auf eine Bewegung der tragbaren Vorrichtung zu einer neuen Zeitzone angepasst wird.

4. System nach Anspruch 1-3, wobei das mindestens eine Beleuchtungselement eine lichtemittierende Diode ist.

5. System nach Ansprüchen 1-3, das geeignet ist, um den circadianen Rhythmus eines Menschen zu synchronisieren, wobei die Steuereinheit konfiguriert ist, um die Intervalle während des Tages auf Grundlage eines 24-Stunden-Zeitplans einzustellen, um den circadianen Rhythmus des Menschen zu synchronisieren.

6. System nach Anspruch 1-3, das geeignet ist, um den circadianen Rhythmus einer Pflanze zu synchronisieren, wobei die Steuereinheit konfiguriert ist, um die Intervalle während des Tages auf Grundlage eines 24-Stunden-Zeitplans einzustellen, um den circadianen Rhythmus der Pflanze zu synchronisieren.

7. System nach Anspruch 1-3, das geeignet ist, um den circadianen Rhythmus eines Tiers zu synchronisieren, wobei die Steuereinheit konfiguriert ist, um die Intervalle während des Tages auf Grundlage eines 24-Stunden-Zeitplans einzustellen, um den circadianen Rhythmus des Tiers zu synchronisieren.

8. System nach Anspruch 1-3, wobei die Steuereinheit konfiguriert ist, um Lichtemission durch das mindestens eine Beleuchtungselement nach einer vorbestimmten Zeit während des Tages zu verhindern.

## Revendications

1. Système servant à entraîner un rythme circadien d'un organisme vivant, comprenant :
un système d'éclairage présentant au moins un élément d'éclairage émettant de la lumière sensiblement concentrée dans une plage comprise entre 360 nm et 400 nm, et
un dispositif de commande présentant un mécanisme de temporisation configuré pour fournir de la lumière sensiblement concentrée dans la plage comprise entre 360 nm et 400 nm à des intervalles de temps pendant une journée ;
dans lequel le dispositif de commande est configuré pour régler les intervalles de temps pendant la journée sur la base d'un système horaire de 24 heures pour entraîner le rythme circadien de l'organisme vivant ;
**caractérisé en ce que**
le dispositif de commande inclut une unité de traitement dotée d'un système de positionnement par satellites, et l'unité de traitement est configurée pour régler le système horaire de 24 heures et les intervalles de temps pendant la journée sur la base de données reçues du système de positionnement par satellites.

2. Système servant à entraîner un rythme circadien d'un organisme vivant, comprenant :
un système d'éclairage présentant au moins un élément d'éclairage émettant de la lumière sensiblement concentrée dans une plage comprise entre 360 nm et 400 nm ; et
un dispositif de commande présentant un mécanisme de temporisation configuré pour fournir de la lumière sensiblement concentrée dans la plage comprise entre 360 nm et 400 nm à des intervalles de temps pendant une journée ;
dans lequel le dispositif de commande est configuré pour régler les intervalles de temps pendant la journée sur la base d'un système horaire de 24 heures pour entraîner le rythme circadien de l'organisme vivant ;
**caractérisé en ce que**
le dispositif de commande inclut une unité de réception configurée pour déterminer une heure locale de données reçues d'un service de temps réseau ; et le dispositif de commande est configuré pour régler le système horaire de 24 heures et les intervalles de temps pendant la journée sur la base de l'heure locale.

3. Système servant à entraîner un rythme circadien d'un organisme vivant, comprenant :
un système d'éclairage présentant au moins un élément d'éclairage émettant de la lumière sensiblement concentrée dans une plage comprise entre 360 nm et 400 nm ; et
un dispositif de commande présentant un mécanisme de temporisation configuré pour fournir de la lumière sensiblement concentrée dans la plage comprise entre 360 nm et 400 nm à des intervalles de temps pendant une journée ;
dans lequel le système d'éclairage est un dispositif portatif, **caractérisé en ce que** le système horaire de 24 heures est réglé en réponse à un mouvement du dispositif portatif vers un nouveau fuseau horaire.

4. Système selon la revendication 1-3,
dans lequel l'au moins un élément d'éclairage est une diode électroluminescente.

5. Système selon les revendications 1-3,
étant adapté pour entraîner le rythme circadien d'un être humain, dans lequel le dispositif de commande est configuré pour régler les intervalles de temps pendant la journée sur la base d'un système horaire de 24 heures pour entraîner le rythme circadien de l'être humain.

6. Système selon la revendication 1-3,
étant adapté pour entraîner le rythme circadien d'une plante, dans lequel le dispositif de commande est configuré pour régler les intervalles de temps pendant la journée sur la base d'un système horaire de 24 heures pour entraîner le rythme circadien de la plante.

7. Système selon la revendication 1-3,
étant adapté pour entraîner le rythme circadien d'un animal, dans lequel le dispositif de commande est configuré pour régler les intervalles de temps pendant la journée sur la base d'un système horaire de 24 heures pour entraîner le rythme circadien de l'animal.

8. Système selon la revendication 1-3,
dans lequel le dispositif de commande est configuré pour prévenir l'émission de lumière par l'au moins un élément d'éclairage après un temps prédéterminé pendant la journée.
